# EUROPEAN PATENT SPECIFICATION

(11) **EP 3 691 508 B1**
(45) Date of publication and mention of the grant of the patent: **07.06.2023**
(21) Application number: 18782736.5
(22) Date of filing: 05.10.2018
(51) Int. Cl.: A61B 1/00, A61B 1/005, A61B 1/015, A61B 1/06

(54) **IMAGING ENDOSCOPE SYSTEM AND ASSOCIATED METHODS**
BILDGEBENDES ENDOSKOPSYSTEM UND ZUGEHÖRIGE VERFAHREN
SYSTÈME D'ENDOSCOPE D'IMAGERIE ET MÉTHODES ASSOCIÉES

(30) Priority: 06.10.2017 GB 201716360; 06.10.2017 GB 201716361
(43) Date of publication of application: 12.08.2020
(73) Proprietor: I.Q. Endoscopes Ltd, Cambridge CB4 0WS (GB)
(72) Inventor: WARD-BOOTH, Patrick, Cambridge CB4 0WS (GB); MILLER, Andrew, Cambridge CB4 0WS (GB); MARTIN, Paul, Cambridge CB4 0WS (GB); FIELD, Stephen, Great Chesterford Saffron Walden CB10 1NH (GB); BRITTON, Polly, Great Chesterford Saffron Walden CB10 1NH (GB)
(74) Representative: Mewburn Ellis LLP
(86) International application number: PCT/EP2018/077187
(87) International publication number: WO 2019/068894

(56) References cited:
- WO-A1-97/28839
- WO-A1-2008/098251
- WO-A1-2010/066787
- WO-A2-2004/086957

## Description

### BACKGROUND TO THE INVENTION

### Field of the invention

The present invention relates to imaging endoscope systems, elements thereof, methods for the assembly of such systems and to methods of operating such systems.

### Related art

It is known that early detection of disease may be assisted by endoscopic examination of internal structures such as the alimentary canals and airways, e. g., the oesophagus, lungs, colon, uterus, and other organ systems. Endoscopic examination may be carried out using an imaging endoscope. Typically, an imaging endoscope has a flexible tube providing a light guide that guides illumination light from an external light source located proximally of the flexible tube to a distal tip of the flexible tube. The illumination light thereby illuminates the tissue to be examined. The distal tip typically also includes an objective lens to gather light from the tissue being examined. The flexible tube provides an imaging light guide to carry the light away from the distal tip and to a camera at the proximal end of the flexible tube. It is also known for the imaging light guide to be dispensed with and replaced with an imaging camera chip at the distal tip. In this case, the signal from the imaging camera chip is conducted along electrical wires in the flexible tube. Based on either approach, an image is produced for display to the imaging endoscope operator.

It is also known, in addition to the imaging function described above, for imaging endoscopes to provide further functionality. For example, it is known for the distal tip to be equipped with a port for dispensing air (or more generally, insufflation gas) to inflate the internal structure being examined, a port for dispensing irrigation liquid such as water or saline, and a port for a medical tool such as biopsy forceps. To allow such services to be provided, the flexible tube typically provides suitable lumens dedicated to the provision of these services.

Although some endoscopic procedures can be carried out with a rigid insertion tube, for many procedures it is preferred that the insertion tube is flexible. This allows the insertion tube to be located along tortuous and often complex paths within the body. To allow adequate steering of the flexible tube within the body, it is known to provide the distal tip of the insertion tube with means for deflecting the distal tip, and therefore with means to steer the distal tip. A typical approach to this is to provide control cables, extending along the flexible tube from a control handle at a proximal end of the flexible tube and anchored to the distal tip. The control handle has control knobs to allow movement of the control wires and consequently steering of the distal tip.

### SUMMARY OF THE INVENTION

The development of imaging endoscopes has to date been based on an assumption that the endoscope will be re-used multiple times. Clearly, where an endoscope is to be used on different individual subjects, it is necessary to thoroughly clean and disinfect the endoscope between uses. The cleaning and disinfecting regime applied to imaging endoscopes is, however, severe. Therefore the imaging endoscope is typically built robustly in order to withstand repeated cycles of cleaning and disinfecting. To an extent, this limits the actual usability of the imaging endoscope and in particular its flexibility. This also tends to mean that imaging endoscopes are expensive items of medical equipment, with costs in the range of tens of thousands of US dollars. Furthermore, it has more recently been considered that the cleaning and disinfecting regime applied to imaging endoscopes is unsatisfactory, leading to a risk of infection when the imaging endoscope is re-used as cleaning the imaging endoscopes enough to create a sterile state is considered impossible.

With these problems in mind, there have been proposals made for single use imaging endoscopes, intended to be used only once and then discarded. As an example of this, WO 2004/086957 discloses a proposal for a single use endoscope.

Document WO-A1-2010/066787 discloses a method for assembling an imaging endoscopy system, the imaging endoscopy system comprising a base unit; a hand controller; an umbilical section releasably connecting the base unit and the hand controller; an insertion section having a proximal end connected to the hand controller and a distal end for insertion into a subject, the distal end having a steering section and a distal tip, wherein the hand controller includes at least one steering control for controlling bending of the steering section, and wherein the distal tip includes a light source for illumination of a region of tissue of interest and an imaging chip for imaging the region of tissue of interest, wherein the method comprises disconnecting a used umbilical section from the base unit, disposing of said used umbilical section and connecting a sterile umbilical section to the base unit to provide an imaging endoscopy system ready for use.

However, the present inventors consider that single-use imaging endoscope systems require further improvement, in order to be suitable for adoption by clinicians and in order to be capable of manufacture at a cost at which disposal after a single use is nevertheless cost-effective.

The present invention has been devised in order to address at least one of the above problems. Preferably, the present invention reduces, ameliorates, avoids or overcomes at least one of the above problems. The present invention is defined by the independent claims.

Accordingly, in a first preferred aspect of a first development, the present invention provides an imaging endoscopy kit for assembling an imaging endoscopy system, the imaging endoscopy system assembled from the kit comprising:
a base unit;
a hand controller;
an umbilical section releasably connecting the base unit and the hand controller;
an insertion section having a proximal end connected to the hand controller and a distal end for insertion into a subject, the distal end having a steering section and a distal tip,

wherein the hand controller includes at least one steering control for controlling bending of the steering section, and wherein the distal tip includes a light source for illumination of a region of tissue of interest and an imaging chip for imaging the region of tissue of interest,
wherein the imaging endoscopy kit comprises:
   the base unit; and
   a plurality of sealed containers, together containing:
      a plurality of said hand controllers, in sterile condition;
      a plurality of said umbilical sections, in sterile condition; and
      a plurality of said insertion sections, in sterile condition.

In a second preferred aspect of the first development, the present invention provides a method for assembling an imaging endoscopy system, the imaging endoscopy system assembled from the kit comprising
a base unit;
a hand controller;
an umbilical section releasably connecting the base unit and the hand controller;
an insertion section having a proximal end connected to the hand controller and a distal end for insertion into a subject, the distal end having a steering section and a distal tip,

wherein the hand controller includes at least one steering control for controlling bending of the steering section, and wherein the distal tip includes a light source for illumination of a region of tissue of interest and an imaging chip for imaging the region of tissue of interest,
wherein the method comprises:
   disconnecting a used umbilical section from the base unit and disposing of said used umbilical section, optionally along with a connected used hand controller and insertion section
   opening at least one sealed container containing:
      a hand controller, in sterile condition;
      an umbilical section, in sterile condition; and
      an insertion section, in sterile condition, and
   connecting the sterile umbilical section to the base unit to provide an imaging endoscopy system ready for use.

The first and/or second aspect of the first development of the invention may have any one or, to the extent that they are compatible, any combination of the following optional features.

Each sealed container may contain one hand controller, one umbilical section and one insertion section. Additionally or alternatively, each hand controller, each umbilical section and each insertion section may be provided in its own respective sealed container.

The hand controller may be releasably connectable to the proximal end of the insertion section.

It is possible for two or more of the hand controller, umbilical section and insertion section to be formed integrally. Thus, these parts of the imaging endoscopy system may not be adapted to be releasably connectable. This allows additional simplification to the assembly of the imaging endoscopy system.

In a third preferred aspect of the first development, the present invention provides an imaging endoscopy system comprising:
a base unit;
a hand controller;
an umbilical section for releasably connecting the base unit and the hand controller;
an insertion section having a proximal end for connection to the hand controller and a distal end for insertion into a subject, the distal end having a steering section and a distal tip,

wherein the hand controller includes at least one steering control for controlling bending of the steering section, and wherein in the distal tip includes a light source for illumination of a region of tissue of interest and an imaging chip for imaging the region of tissue of interest,
wherein the system is adapted to provide a flow of irrigation liquid to the region of tissue of interest from the base unit, along the umbilical section, through the hand controller and along the insertion section,
the base unit has an irrigation liquid valve, control of the irrigation liquid valve controlling said flow of irrigation liquid, and
the hand controller has an irrigation liquid electronic control switch, operation of the irrigation liquid electronic control switch at the hand controller controlling the irrigation liquid valve.

In a fourth preferred aspect of the first development, the present invention provides a method of operating an imaging endoscopy system, the imaging endoscopy system comprising:
a base unit;
a hand controller;
an umbilical section for releasably connecting the base unit and the hand controller;
an insertion section having a proximal end for connection to the hand controller and a distal end for insertion into a subject, the distal end having a steering section and a distal tip,

wherein the hand controller includes at least one steering control for controlling bending of the steering section, and wherein in the distal tip includes a light source for illumination of a region of tissue of interest and an imaging chip for imaging the region of tissue of interest,
the method including:
   providing a flow of irrigation liquid to the region of tissue of interest from the base unit, along the umbilical section, through the hand controller and along the insertion section, operating an irrigation liquid electronic control switch at the hand controller to control an irrigation liquid valve in the base unit and thereby to control said flow of irrigation liquid.

The third and/or fourth aspect of the first development of the invention may have any one or, to the extent that they are compatible, any combination of the following optional features and/or the optional features set out with respect to the first and/or second aspect of the first development of the invention. The following optional features may also be applied to the first and/or second aspect of the first development of the invention.

The system may be adapted to provide a flow of insufflation gas to the region of tissue of interest from the base unit, along the umbilical section, through the hand controller and along the insertion section. The base unit may therefore have an insufflation gas valve, control of the insufflation gas valve controlling said flow of insufflation gas. The hand controller may therefore have an insufflation gas electronic control switch, operation of the insufflation gas electronic control switch at the hand controller controlling the insufflation gas valve.

The system may be adapted to provide suction to the region of tissue of interest, via the insertion section. The system may therefore further comprise a vacuum source. The base unit may have a suction control valve connected to the vacuum source. The hand controller may have an electronic suction control switch, operation of the electronic suction control switch at the hand controller controlling the suction control valve to control the provision of suction to the region of tissue of interest.

In some embodiments, it is intended that each switch provides on-off control only. This allows for simple design of the system. Suitable switches can be implemented cost effectively.

In some embodiments, one or more of the switches can be implemented using a touch-sensitive apparatus. For example, a touchpad or swipe pad may be provided.

The hand controller may have an imaging operation switch, adapted to control the system to record at least one of moving images and still images.

During operation of the system, there may be provided a flow of insufflation gas to the region of tissue of interest from the base unit, along the umbilical section, through the hand controller and along the insertion section. Control of the insufflation gas valve controls said flow of insufflation gas. Operation of the insufflation gas electronic control switch at the hand controller may therefore control the insufflation gas valve.

During operation of the system, there may be provided suction to the region of tissue of interest, via the insertion section. A suitable vacuum source may be a vacuum bottle. The suction control valve connected to the vacuum source may therefore be controlled by the electronic suction control switch, in order to control the provision of suction to the region of tissue of interest.

The preferred embodiments of the invention provide a single reusable base unit for sequential use with several interchangeable identical endoscope components (comprising the umbilical section, hand controller and insertion section, for example). One of the primary reasons for adopting this approach is that it is then possible to provide the interchangeable endoscope components in sterile condition at the time of attachment to the base unit. After a single use, the interchangeable endoscope component is intended to be detached from the base unit, disposed of, and the next interchangeable endoscope component, in sterile condition, attached to the base component ready for use.

It is desirable to ensure that one of the used (and therefore non-sterile) interchangeable endoscope components cannot be re-attached to the base unit for a subsequent use. Such re-attachment may be done through error or as a deliberate act. In order to provide a barrier to this occurrence, it is preferred to equip the system with means to prevent the base unit from servicing an interchangeable endoscope component that has been previously attached and used on the same base unit.

Accordingly, in a fifth aspect of the first development, the present invention provides an imaging endoscopy kit for assembling an imaging endoscopy system, the imaging endoscopy system assembled from the kit comprising:
a base unit;
a hand controller;
an umbilical section releasably connecting the base unit and the hand controller;
an insertion section having a proximal end connected to the hand controller and a distal end for insertion into a subject, the distal end having a steering section and a distal tip,

wherein the hand controller includes at least one steering control for controlling bending of the steering section, and wherein the distal tip includes a light source for illumination of a region of tissue of interest and an imaging chip for imaging the region of tissue of interest,
the imaging endoscopy system having a single use confirmation system in which the base unit has a first radio frequency identification means and the umbilical section has a second radio frequency identification means, the single use confirmation system permitting the imaging endoscopy system to operate only when it confirms, based on interaction between the first and second radio frequency identification means that the umbilical section has not previously been used with the base unit.

In a sixth aspect of the first development, the present invention provides a method for assembling an imaging endoscopy system, the imaging endoscopy system comprising:
a base unit;
a hand controller;
an umbilical section releasably connecting the base unit and the hand controller;
an insertion section having a proximal end connected to the hand controller and a distal end for insertion into a subject, the distal end having a steering section and a distal tip,

wherein the hand controller includes at least one steering control for controlling bending of the steering section, and wherein the distal tip includes a light source for illumination of a region of tissue of interest and an imaging chip for imaging the region of tissue of interest,
wherein the method comprises:
   disconnecting a used umbilical section from the base unit and disposing of said used umbilical section, optionally along with a connected used hand controller and insertion section;
   providing an unused sterile umbilical section optionally along with a connected used hand controller and insertion section; and
   connecting the unused sterile umbilical section to the base unit to provide an imaging endoscopy system ready for use,
   the imaging endoscopy system having a single use confirmation system in which the base unit has a first radio frequency identification means and the unused sterile umbilical section has a second radio frequency identification means, the single use confirmation system permitting the imaging endoscopy system to operate only when it confirms, based on interaction between the first and second radio frequency identification means that the unused sterile umbilical section has not previously been used with the base unit.

The single use confirmation system may be an active radio frequency identification system (ARFID) system or a Bluetooth low energy (BLE) system, for example. Such systems provide reliable operation and can be implemented using established standard communications protocols.

The base unit typically provides electrical power, irrigation liquid and insufflation gas to the insertion section, via the umbilical section and hand controller. It is preferred that the single use confirmation system is operable to prevent one, more than one or all of supply of electrical power, irrigation liquid and insufflation gas when the single use confirmation system detects that the umbilical section has previously been used with the base unit. This renders the imaging endoscopy system inoperable and provides a substantial barrier to the accidental or deliberate re-use of the disposable components of the imaging endoscopy system.

In a first preferred aspect of a second development, the present invention provides an insertion section for an imaging endoscopy system, the insertion section having a proximal end for connection to a hand controller and a distal end for insertion into a subject, the distal end having a steering section and a distal tip, wherein the insertion section has a core part and an outer part, the outer part surrounding the core part, the core part being formed as an extruded member having a cross sectional shape providing at least two integral internal lumens extending along the interior of the core part from the proximal end to the distal end.

In a second preferred aspect of the second development, the present invention provides an insertion section for an imaging endoscopy system, the insertion section having a proximal end for connection to a hand controller and a distal end for insertion into a subject, the distal end having a steering section and a distal tip, wherein in the distal tip includes a light source for illumination of a region of tissue of interest and an imaging chip for imaging the region of tissue of interest.

The first and/or second aspect of the second development of the invention may have any one or, to the extent that they are compatible, any combination of the following optional features and/or the optional features set out with respect to any aspect of the first development of the invention. The following optional features may also be applied to any aspect of the first development of the invention.

There may be provided at least three integral internal lumens extending along the interior of the core part from the proximal end to the distal end. There may be provided at least four, five, six, seven, eight or nine integral internal lumens extending along the interior of the core part from the proximal end to the distal end.

The lumens may be provided substantially equiangularly spaced around the insertion section when viewed in cross section.

At least one lumen may be designated for use to conduct a flow of irrigation liquid to the distal tip.

At least one lumen may be designated for use to conduct a flow of insufflation gas to the distal tip.

At least one lumen may be designated for use to provide suction at the distal tip.

At least one lumen may be designated for use to conduct a surgical instrument to the distal tip.

At least one lumen may be located along a central axis of the core part.

The insertion section may further comprise at least one steering wire, extending from the proximal end for connection to the hand controller to the distal tip. The steering wire may be located interiorly of the outer part of the insertion section. The steering wire is preferably located between the core part and the outer part of the insertion section.

There may be provided four steering wires, substantially equiangularly spaced around the insertion section when viewed in cross section.

The steering wires may have sheaths each defining an axis of constrained movement for each respective steering wire. Preferably, the steering wires extend through the steering section to the distal tip and the sheaths of the steering wires do not extend through the steering section.

The steering section may have a series of links pivotably connected to each other, the links arranged to cooperate to permit controlled bending of the steering section. Preferably, pivotable connections between each link provides a limit to the range of relative movement between adjacent links. The links may have apertures dimensioned to receive and conduct the steering wires.

The core part may extend through the steering section to the distal tip. This is in contrast to the sheaths of the steering wires, in some embodiments.

Preferably, the distal tip includes a light source for illumination of a region of tissue of interest. There may be provided at least one electrical conductor along the insertion section for providing electrical power to the light source.

Preferably, the distal tip includes an imaging chip for imaging the region of tissue of interest. There may be provided at least one electrical conductor for conducting electrical signals from the imaging chip to the proximal end of the insertion section.

In this manner, it is possible to implement an imaging endoscope system in which there is no need for the insertion section to transmit light, whether as illumination or optical images.

Preferably, the light source includes at least one light emitting diode (LED).

The distal tip may include a distal tip housing which is integrally formed from light-transmissive material. The distal tip housing may be integrally formed from plastics material. In operation, the distal tip housing may diffuse illumination light from the light source. This can be helpful in providing a satisfactory and consistent illumination field for imaging.

The distal tip housing may have a collar portion extending proximally of a distal end face. The collar portion may be adapted to fit over a distal end of the steering section. This is considered to be a convenient implementation.

The distal tip housing may have at least one lumen extension portion. This may be adapted to engage with a corresponding lumen extending along a core part of the insertion section. The lumen extension portion may open to a distal end face of the distal tip housing.

The distal tip housing may have at least two lumen extension portions. These may be adapted to engage with respective corresponding lumens extending along a core part of the insertion section. The lumen extension portions may be substantially equiangularly spaced around the distal tip housing when viewed in plan view.

The distal tip housing has a cleaning nozzle arranged at the distal end face, to direct irrigation liquid to clean a lens of the imaging chip.

In a third aspect of the second development, the invention provides an imaging endoscopy system comprising:
a hand controller;
an insertion section having a proximal end connected to the hand controller and a distal end for insertion into a subject, the distal end having a steering section and a distal tip; at least one steering wire, extending from the hand controller to the distal tip, the steering wire capable of being tensioned to cause bending of the steering section; and at least one rotational drive wheel mounted for rotation at the hand controller and configured to apply tension to the steering wire according to a rotation of the rotational drive wheel applied by a user to bend the steering section and thereby steer the distal tip, wherein the rotational drive wheel, for at least part of its rotation, applies a non-linear length change to the steering wire per angle of rotation.

Preferably, the steering wire follows a steering wire path around the rotational drive wheel, and wherein the steering wire path is non-circular for at least part of the steering wire path. The rotational drive wheel may be mounted for rotation about an axis which is offset from a centre of the rotational drive wheel. For example, the rotational drive wheel may be mounted for rotation about an axis which is offset proximally from a centre of the rotational drive wheel.

In this manner, the steering wire and the rotational drive wheel can be configured so that when the steering section is straight (at least in the plane of movement caused by the steering wire under consideration), rotation of the rotational drive wheel allows relatively fine control over the movement of the distal tip per unit angle of rotation of the rotational drive wheel. In contrast, when the steering section is already bent, there is provided a greater length change of the steering wire per unit angle of rotation of the rotational drive wheel. This allows relatively gross control over the movement of the distal tip.

In a fourth aspect of the second development, the invention provides an imaging endoscopy system comprising:
a hand controller;
an insertion section having a proximal end connected to the hand controller and a distal end for insertion into a subject, the distal end having a steering section and a distal tip; at least a first pair of steering wires, extending from the hand controller to the distal tip, the steering wires capable of being tensioned to cause bending of the steering section; and
at least a first rotational drive wheel mounted for rotation at the hand controller and configured to apply tension to the first pair of steering wires according to a rotation of the first rotational drive wheel applied by a user to bend the steering section and thereby steer the distal tip in a first plane,
wherein the first pair of steering wires is formed of a continuous wire extending from the steering section to wrap at least partially around the first rotational drive wheel and extend back to the steering section.

The imaging endoscopy system may further comprise:
a second pair of steering wires, extending from the hand controller to the distal tip, the steering wires capable of being tensioned to cause bending of the steering section; and
at least a second rotational drive wheel mounted for rotation at the hand controller and configured to apply tension to the second pair of steering wires according to a rotation of the second rotational drive wheel applied by a user to bend the steering section and thereby steer the distal tip in a second plane, perpendicular to the first plane
wherein the second pair of steering wires is formed of a continuous wire extending from the steering section to wrap at least partially around the second rotational drive wheel and extend back to the steering section.

The use of continuous steering wires improves the simplicity of the design of the system, providing a convenient manner to accommodate the required length changes for the steering wire due to the bending applied to the steering section.

Reusable endoscopes are cleaned between uses through a high level disinfection process. This process entails the use of dedicated cleaning equipment which cleans the external surfaces of the endoscope and the internal air, water and vacuum channels with highly aggressive cleaning fluids in a washing machine designed for this purpose. This process necessitates that the reusable endoscope is sealed to ensure that the aggressive cleaning fluids do not enter the internal mechanism of the endoscope. Therefore reusable endoscopes are designed to be impenetrable to the aggressive cleaning fluids. This is very difficult to achieve and is not always successful in practice which leads to premature breakdown of reusable endoscopes due to damage to the internal mechanism by the aggressive cleaning fluids.

With the provision of single use disposable endoscopes according to preferred embodiments of the invention, the present inventors have realised that the accepted design requirements for reusable endoscopes do not apply to this new field of disposable endoscopes. It is therefore not necessary to design a disposable endoscope to survive repeated (or even a single) washing and disinfection process. This is counter-intuitive, but allows the disposable endoscope to have a much simplified design and construction, which has significant benefits on the cost of production of the endoscope.

Accordingly, in a first aspect of a third development of the present invention, there is provided an imaging endoscopy system comprising:
a base unit;
a hand controller;
an umbilical section releasably connecting the base unit and the hand controller;
an insertion section having a proximal end connected to the hand controller and a distal end for insertion into a subject, the distal end having a steering section and a distal tip, the distal tip including a light source for illumination of a region of tissue of interest and an imaging chip for imaging the region of tissue of interest,

wherein the hand controller includes at least one steering control for controlling bending of the steering section, the steering control being operable to control the length of steering wires between the steering control and the steering section, the steering wires passing along steering channels in the insertion section,
wherein, when the umbilical section is detached from the base unit and the hand controller and insertion section are immersed in water at room temperature and pressure, the hand controller and/or the insertion section is water permeable to permit ingress of water into the steering channels in the insertion section.

For a conventional reusable imaging endoscope having steerability, it would be inconceivable for it to be designed to allow water ingress in the manner permitted by embodiments of the present invention. In particular, having water (or other fluid substances) ingress into the steering channels would be highly undesirable, in view of the difficulty in satisfactorily cleaning the steering channels. However, with the single use disposable approach used in the preferred embodiments of the present invention, such ingress is acceptable, because subsequent cleaning of the disposable components of the endoscope system is not required.

Known reusable endoscopes have an insertion section providing a core part for the various service requirements of the endoscope - insufflation gas, suction, surgical tool access, irrigation liquid, steering wires, power and signals. Surrounding this core part is an outer part, which is intended to protect the services in the core part, seal the core part and provide torsional rigidity to the insertion section while allowing bending flexibility, to allow the insertion section to be advanced into the body without causing damage.

Typically, in such reusable endoscopes, the outer part is formed of one or two helically wound flat springs which are essentially tubular. These are over-braided with a braided fibre layer and then an outer smooth sleeve is extruded over the braid. This construction can provide a good bending performance - typically an approximately 13.00mm outer diameter (OD) colonoscope can be bent to an inner radius of approximately 90mm. This is required to negotiate the inside of the colon during insertion and retraction. As well as being able to reach a very small bending radius, the colonoscope must also be able to bear high torsional loads of approximately 0.1 deg/Nm² in order that the medical practitioner can exert a rotation to the distal tip of the endoscope when inserted into the patient.

The present inventors have found that, for the purposes of a single use disposable endoscope system, the insertion section may use a different outer construction. In particular, it has been found that the helically wound flat springs are not necessary. They can be replaced with a ridged tube. The ridges typically run circumferentially around the tube, in a direction perpendicular to the principal axis of the insertion section. It is found that such a construction can provide suitable bending flexibility in combination with torsional rigidity.

Accordingly, in a second aspect of the third development, the present invention provides an insertion section for an imaging endoscopy system, the insertion section having a proximal end for connection to a hand controller and a distal end for insertion into a subject, the distal end having a steering section and a distal tip, wherein the insertion section has a core part and an outer part, the outer part surrounding the core part, wherein the outer part comprises:
a ridged inner tube, an outer face of the ridged inner tube having a circumferential ridged configuration and an inner face of the ridged inner tube having a non-ridged configuration; and
an outer reinforcing construction.

The outer reinforcing construction may comprise a braided fibre layer, overlying the ridged inner tube. Furthermore, the outer reinforcing construction may further comprise a smooth outer layer, overlying the braided fibre layer.

In the present disclosure, it is intended that the "sterile condition" of the endoscope components meets typical requirements for medical devices. Accordingly, it is preferred that the components meet a sterility assurance level (SAL) of 10⁻⁶. At the time of writing, this is a widely-accepted sterility assurance level, corresponding to the probability of non-sterility.

Further optional features of the invention are set out below.

### BRIEF DESCRIPTION OF THE DRAWINGS

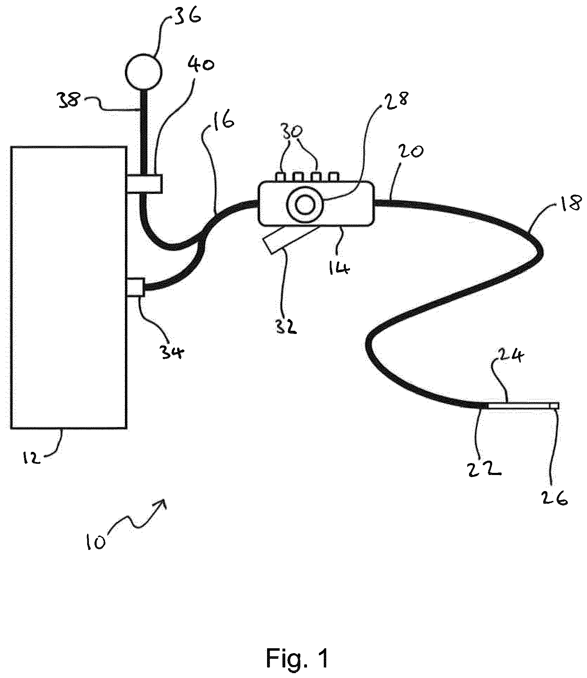

Embodiments of the invention will now be described by way of example with reference to Fig. 1 shows a schematic view of an imaging endoscopy system according to an embodiment of the present invention.
Fig. 2 shows a schematic view of insertion sections suitable for use in different formats of imaging endoscope.
Figs. 3 and 4 show cross sectional view of the insertion sections of Fig. 2.
Fig. 5 shows a side view of a steering section for use with an embodiment of the invention.
Fig. 6 shows a magnified view of the distal end of the steering section of Fig. 5.
Fig. 7 shows a perspective view of the steering section of Fig. 5 assembled on the insertion section.
Fig. 8 shows a magnified view of the steering section of Fig. 7, with the distal tip shown in outline.
Fig. 9 shows a rear perspective view of a distal tip for use with an embodiment of the invention.
Fig. 10 shows the distal tip of Fig. 9 in plan view.
Fig. 11 shows a schematic cross sectional view through a circuit board in the distal tip, with the distal tip shown in outline.
Fig. 12 shows the circuit board of Fig. 11 in plan view.
Fig. 13 shows the circuit board of Fig. 11 in rear perspective view.
Fig. 14 shows the circuit board of Fig. 11 in front perspective view.
Fig. 15 shows the distal tip in rear plan view.
Fig. 16 shows the distal tip in side view.
Fig. 17 shows the distal tip in front plan view.
Fig. 18 shows the distal tip in front perspective view.
Fig. 19 shows the hand controller in exploded view.
Fig. 20 shows one half of the hand controller in exploded view.
Fig. 21 shows another half of the hand controller in exploded view.
Fig. 22 shows part of the hand controller in top view.
Fig. 23 shows part of the hand controller in side view.
Fig. 24 shows part of the hand controller in rear view.
Figs. 25A-25E show schematically the process for swapping replacement interchangeable endoscope components according to an embodiment of the invention.
Fig. 26 shows a schematic partial view of attachment of an insertion section to a base unit according to an embodiment of the invention.
Figs. 27A and 27B show examples of rotational drive wheels providing non-linear length change of the steering wires per angle of rotation.
Fig. 28 shows a partial cross sectional view of an insertion section for use with an embodiment of the invention.

### DETAILED DESCRIPTION OF THE PREFERRED EMBODIMENTS, AND FURTHER OPTIONAL FEATURES OF THE INVENTION

A flexible imaging endoscope is a device that provides images of the inside of the body. It is a fundamental piece of apparatus used within clinical operations; and is used to look for, or exclude, a wide range of disorders, including the early detection of cancers. Currently, endoscopes are expensive reusable instruments, which must be sterilised after every use to avoid cross-contamination between patients. They are difficult to decontaminate, and must be serviced properly to maintain reliable operation.

The preferred embodiments of the present invention provide single-use disposable endoscopes which aims to reduce or remove these problems, at a cost which is below the current capital costs of purchase of conventional imaging endoscopes, when the costs for decontamination, maintenance and repair are taken into account.

The advantages of the preferred embodiments over conventional reusable endoscope systems include the elimination of the risk of cross infections. As suggested above, the preferred embodiments can reduce the cost of endoscopy to less than the current cost of purchasing, cleaning and maintaining reusable devices. The preferred embodiments allow the opening of emerging and developing economies to the endoscopy market.

The basis of the preferred embodiments is to provide a reusable base unit along with a plurality of interchangeable (typically identical) endoscope components. One of the primary reasons for adopting this particular concept is that the sterility of the endoscope components can be assured at the time of manufacture and packaging. Current reusable endoscopes do not meet these standards of sterility as they can only be subjected to a high level disinfection process between uses. This process does not produce sterile endoscope components and unfortunately has been reported to have led to cross-infection between patients, leading to serious illness and in some cases death. In contrast, the interchangeable endoscope components disclosed here can be manufactured and packaged using clean room facilities thus ensuring that the necessary requirements for sterility are met.

The preferred embodiments of the invention have been designed with low complexity for ease of manufacture, and to take advantage of the disposable nature of the instrument to reduce costs on materials and features that do not need to survive repeated cleaning and use.

Fig. 1 shows a schematic view of an imaging endoscopy system 10 according to an embodiment of the present invention. The system includes a base unit 12 that is intended to be re-usable. Umbilical section 16 releasably connects the base unit 12 and hand controller 14. Insertion section 18 has a proximal end 20 connected to the hand controller 14 and a distal end 22 for insertion into a subject. The distal end 22 has a steering section 24 and a distal tip 26.

The hand controller 14 includes at least one steering control 28, described in more detail later, for controlling bending of the steering section 24. The hand controller 14 further includes switches 30, whose operation is described later. The hand controller 14 has a tool insertion port 32.

Umbilical section 16 attaches to base unit 12 via a quick release connector 34.

Vacuum source 36 is typically a vacuum bottle as already known for medical applications. Vacuum line 38 extends from vacuum source 36 through pinch valve 40. The operation of pinch valve 40 is described later, but the advantage of a pinch valve is that the valve acts on external surface of the vacuum line and so can be re-used without risk of contamination between procedures.

Distal tip 26 includes a light source (typically an LED, described later) for illumination of a region of tissue of interest. Distal tip 26 further includes an imaging chip (described later) for imaging the region of tissue of interest.

In kit form, the system comprises the base unit 12, typically a single base unit 12, and a plurality of sealed containers. The plurality of sealed containers together contain a plurality of hand controllers 14, in sterile condition, a plurality of umbilical sections 16, in sterile condition, and a plurality of insertion sections 18, in sterile condition.

In some embodiments, the hand controller 14 and the umbilical section 16 and the insertion section 18 may be formed integrally in the sense that they are assembled together in the factory, and contained together in a sealed container (described later).

For assembly of the kit, the base unit 12 is located in a suitable location for an endoscopic procedure. Where a preceding endoscopic procedure has been carried out, any previously-used umbilical section 16 is disconnected from the base unit 12 and disposed of. Typically, a used connected used hand controller and insertion section are also disposed of.

To ready the kit for the endoscopic procedure, an operator (an endoscopist, or an assistant) opens at least one of said sealed containers, extracts the hand controller 14, in sterile condition, the umbilical section 16, in sterile condition, and the insertion section 18, in sterile condition. The sterile umbilical section 16 is then connected to the base unit 12 to provide an imaging endoscopy system 10 ready for use.

After use, the hand controller 14, the umbilical section 16 and the insertion section 18 will be contaminated. Additionally, the vacuum line 38 and vacuum source 36 will be contaminated. However, rather than subjecting these elements of the system to cleaning and attempting to sterilize them, they are disposed of after a single use. This allows these elements of the system to have a simpler structure than is conventional, since they need not withstand repeated cleaning operations.

Each sealed container may contain one hand controller, one umbilical section and one insertion section. Additionally or alternatively, each hand controller, each umbilical section and each insertion section may be provided in its own respective sealed container.

The insertion section 18 will now be described in more detail.

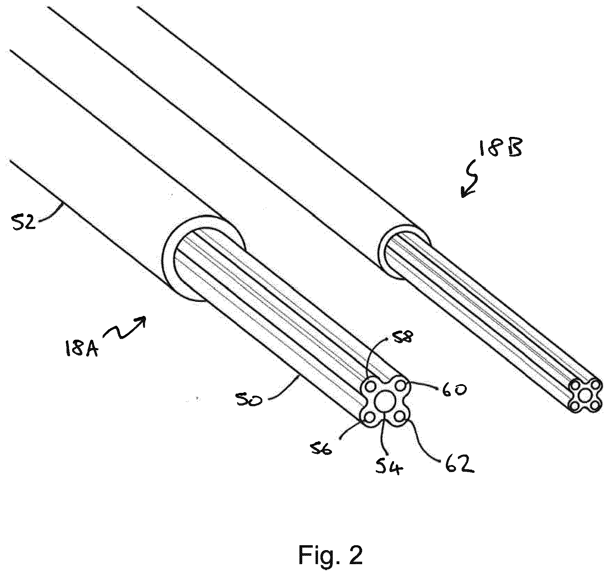

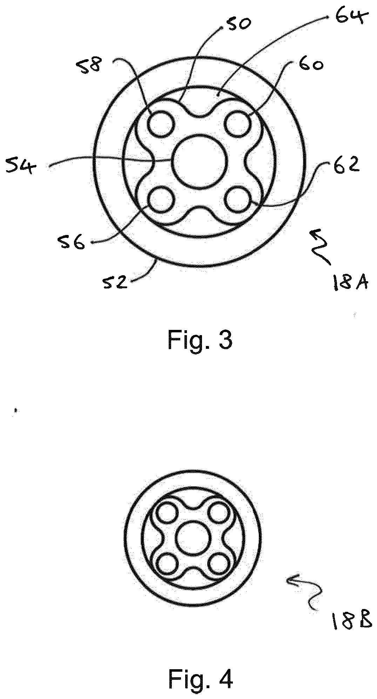

Fig. 2 shows a schematic view of insertion sections suitable for use in different formats of imaging endoscope. Insertion section 18A has a relatively large format, suitable for use for example in colonoscopy. Insertion section 18B in contrast has a relatively smaller format, suitable for use for example in gastroscopy. Figs. 3 and 4 show cross sectional view of the insertion sections 18A and 18B of Fig. 2.

As shown in Fig. 1, the insertion section 18 has a proximal end 20 for connection to the hand controller 14 and a distal end 22 for insertion into a subject. The distal end 22 has a steering section 24 and a distal tip26.

Taking insertion section 18A as an example, the insertion section has a core part 50 and an outer part 52, the outer part 52 surrounding the core part 50. The core part 50 is formed as an extruded member. The cross sectional shape of the core part 50 provides, in this embodiment, five integral internal lumens 54, 56, 58, 60, 62 extending along the interior of the core part 50 from the proximal end to the distal end. Central lumen 54 has a relatively large diameter, adapted to conduct a surgical instrument such as biopsy forceps and/or to provide suction. Peripheral lumens 56, 58, 60, 62 are equiangularly spaced around the central lumen 54.

For example, lumen 56 may be designated for use to conduct a flow of irrigation liquid to the distal tip, lumen 58 may be designated for use to conduct a flow of insufflation gas to the distal tip, lumen 60 may be designated to conduct one or more electrical conductors to provide power to the distal tip.

The insertion section further comprises steering wires (not shown), extending from the proximal end 20 for connection to the hand controller 14 to the distal tip 26. The steering wire are located interiorly of the outer part 52 of the insertion section 18. The steering wire is located between the core part 50 and the outer part 52 of the insertion section 18.

As shown most clearly in Fig. 3, the shape of the core part 50 is such that an external surface of the core part has an undulating shape to accommodate the peripheral lumens 56, 58, 60, 62. When surrounded by the outer part 52, there are therefore axially extended spaces 64 located between the core part 50 and the outer part 52. In these spaces are located the sheathed steering wires (not shown). Thus, the embodiments of Figs. 2-4 allow for four steering wires, substantially equiangularly spaced around the insertion section 18 when viewed in cross section.

The sheaths of the steering wires define an axis of constrained movement for each respective steering wire. Such an arrangement is typically referred to as a Bowden cable. The steering wires extend through the steering section 24 to the distal tip 26 and the sheaths of the steering wires do not extend through the steering section 24.

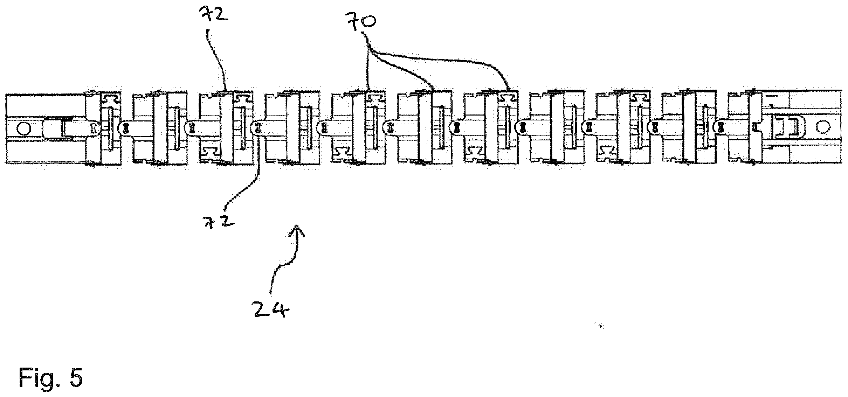

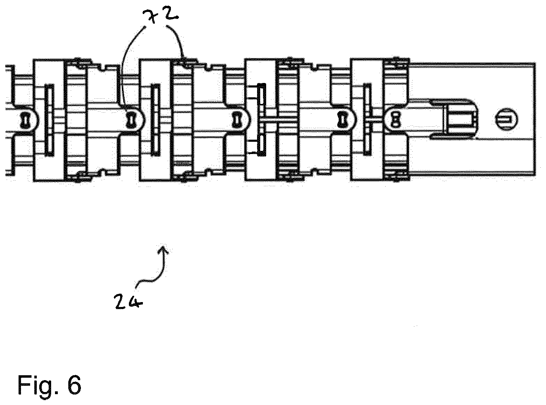

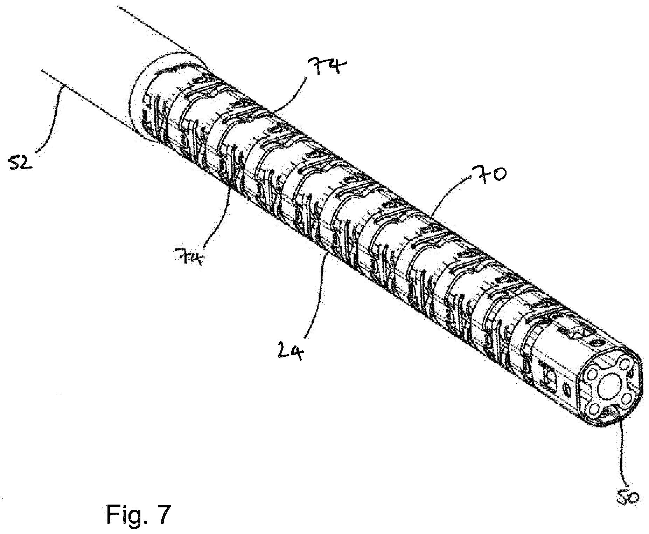

Fig. 5 shows a side view of a steering section 24 for use with an embodiment of the invention. Fig. 6 shows a magnified view of the distal end of the steering section of Fig. 5. Fig. 7 shows a perspective view of the steering section of Fig. 5 assembled on the insertion section.

As shown in Fig. 5, the steering section has a series of links 70 pivotably connected to each other. The links 70 are connected by hinges 72 permitting only limited pivoting between adjacent links. Each link 70 may be formed by stamping and bending of sheet metal. Together, the links and hinges allow for two axis controlled bending of the steering section. The links 70 have apertures 74 dimensioned to receive and conduct the steering wires. These apertures therefore together provide a constrained path for the steering wires, allowing the steering wire sheaths to terminate proximally of the steering section.

The core part 50 extends through the steering section 24 to the distal tip 26. This is in contrast to the sheaths of the steering wires, in the preferred embodiment.

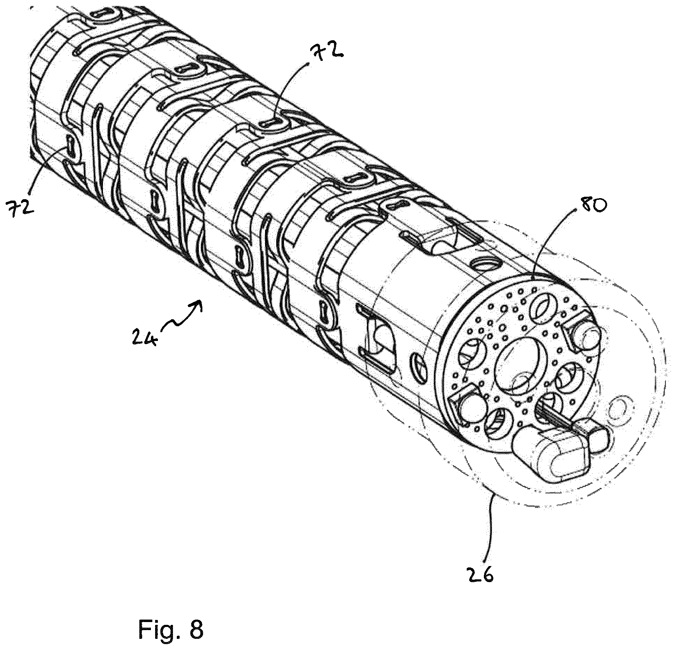

Fig. 8 shows a magnified view of the steering section 24 of Fig. 7, with the distal tip 26 shown in outline. This shows how the steering section and the distal tip cooperate.

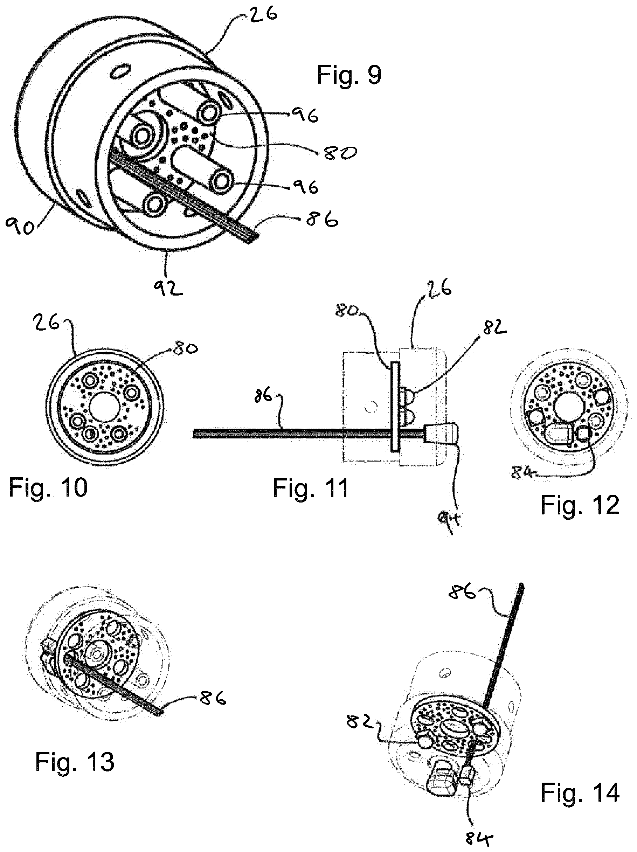

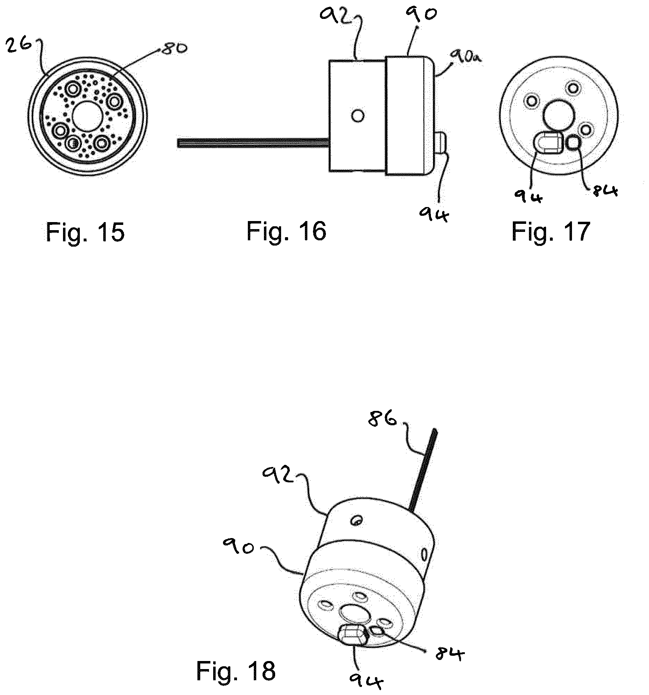

Fig. 9 shows a rear perspective view of the distal tip 26. Fig. 10 shows the distal tip of Fig. 9 in plan view. Fig. 11 shows a schematic cross sectional view through a circuit board 80 in the distal tip, with the distal tip 26 shown in outline. Fig. 12 shows the circuit board of Fig. 11 in plan view. Fig. 13 shows the circuit board of Fig. 11 in rear perspective view. Fig. 14 shows the circuit board of Fig. 11 in front perspective view. Fig. 15 shows the distal tip in rear plan view. Fig. 16 shows the distal tip in side view. Fig. 17 shows the distal tip in front plan view. Fig. 18 shows the distal tip in front perspective view.

The distal tip 26 includes a light source 82 for illumination of a region of tissue of interest. In the embodiment, there are shown two light sources 82 and board-mounted LEDs. At least one electrical conductor is provided along the insertion section 18 for providing electrical power to the light source 82.

The distal tip 26 includes an imaging chip 84 for imaging the region of tissue of interest. Imaging chip 84 is provided in the form of a camera, with an objective lens located to collect and direct light onto the imaging chip. Electrical conductor 86 is provided for conducting electrical signals from the imaging chip 84 to the proximal end 20 of the insertion section 18.

In this manner, it is possible to implement an imaging endoscope system in which there is no need for the insertion section 18 to transmit light, whether as illumination or optical images.

The distal tip 26 may include a distal tip housing 90 which is integrally formed from light-transmissive material. For example, distal tip housing 90 may be formed by injecting moulding of suitable light-transmitting plastics material. In operation, the distal tip housing 90 may diffuse illumination light from the light source 82. This can be helpful in providing a satisfactory and consistent illumination field for imaging.

The distal tip housing 90 has a collar portion 92 extending proximally of a distal end face 90a. The collar portion 92 is adapted to fit over a distal end of the steering section 18. The distal tip housing 90 has four lumen extension portions 96. These are adapted to engage with corresponding lumens 56, 58, 60, 62 extending along the core part 50 of the insertion section 18. The lumen extension portions 96 open to the distal end face 90a of the distal tip housing 90.

The distal tip housing 90 has a cleaning nozzle 94 arranged at the distal end face 90a, to direct irrigation liquid to clean a lens of the imaging chip 84.

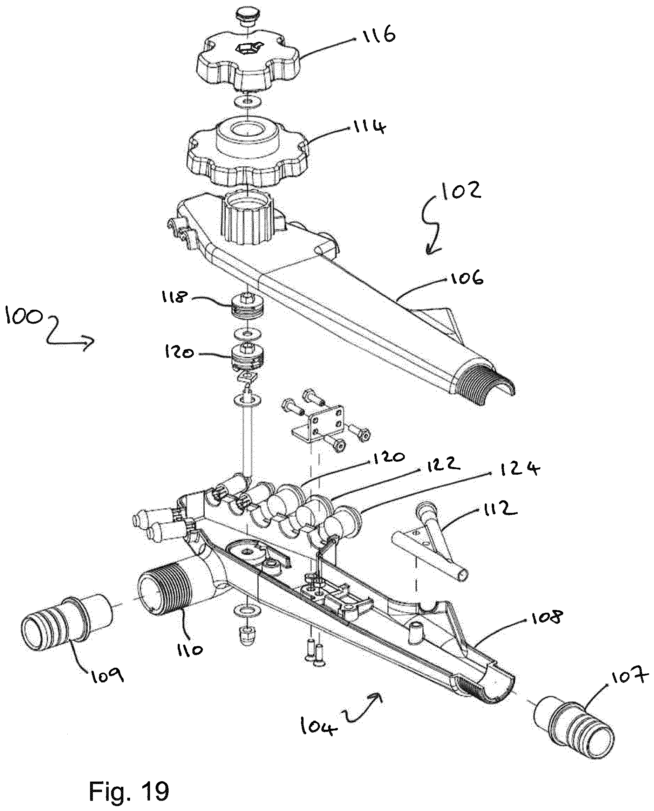

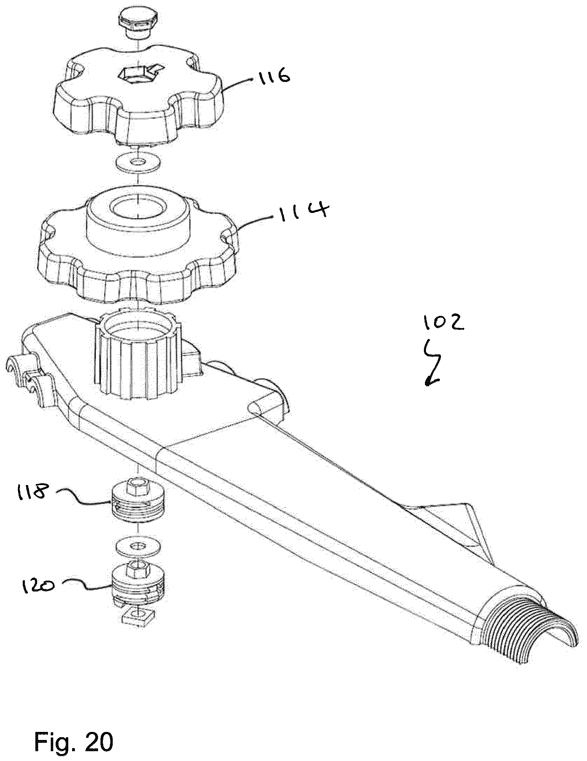

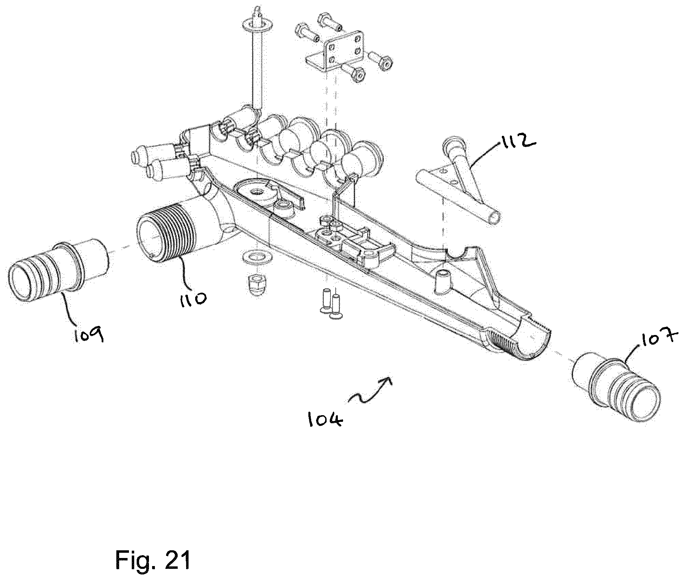

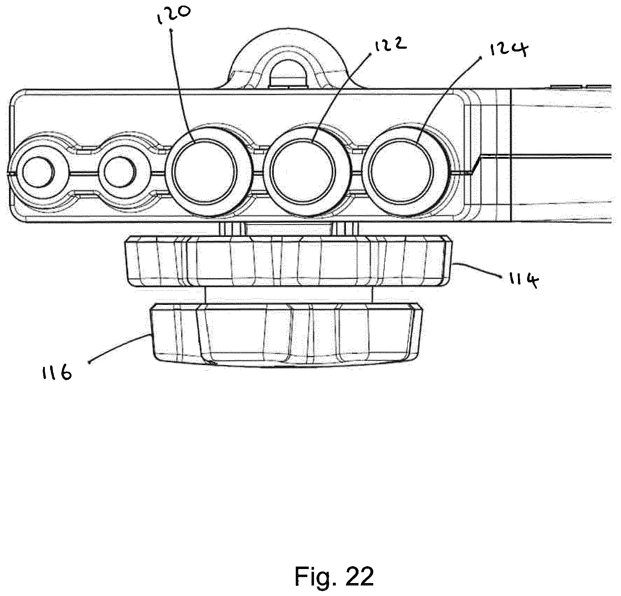

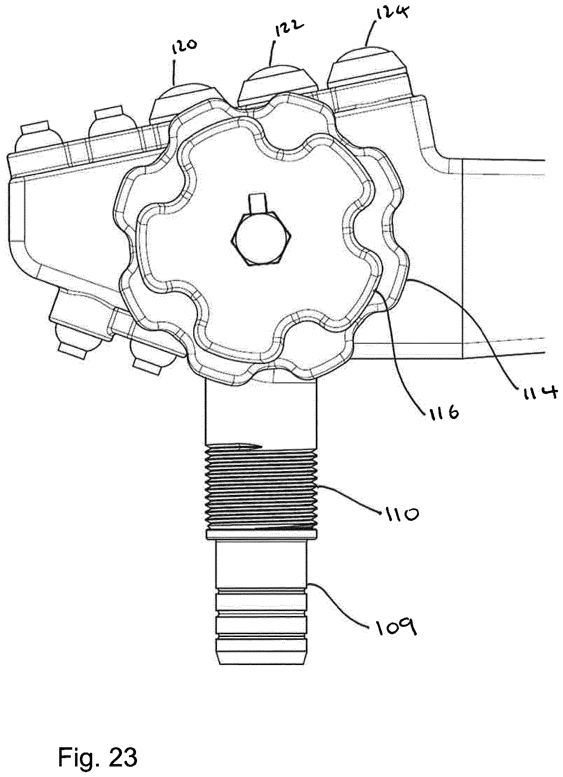

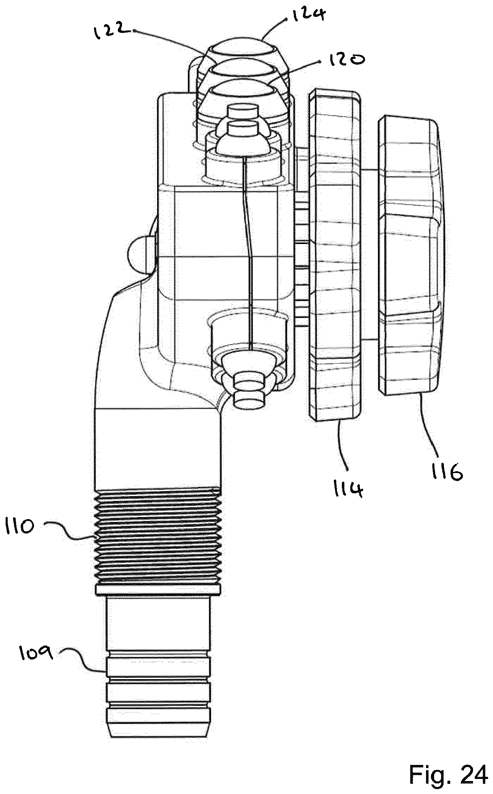

Fig. 19 shows the hand controller 14, 100 in exploded view. Fig. 20 shows one half 102 of the hand controller in exploded view. Fig. 21 shows another half 104 of the hand controller in exploded view. Fig. 22 shows part of the hand controller in top view. Fig. 23 shows part of the hand controller in side view. Fig. 24 shows part of the hand controller in rear view. Where appropriate, corresponding parts in the different drawings use the same reference numbers and are not described in relating to each drawing.

The hand controller is formed of two main injection moulded parts, designated as side part 106 and side part 108. Side parts 106 and side part 108 attach to each other along a longitudinal join and cooperate to form a distal connector for attachment to proximal end 20 of the insertion section 18 via connector 107. Side part 108 provides proximal connector 110 for attachment to distal end 20 of the umbilical section 16 via connector 109.

Surgical instrument port 112 is provided for incorporation in a base side of the hand controller.

Steering wire controls 114, 116 are provided externally on the hand controller, for controlling steering wire control wheels 118, 120. The arrangement of the steering wire controls 114, 116 is adapted to be similar to known imaging endoscope systems, in order for the operation of the present system by an endoscopist to be as intuitive as possible. Turing of the steering wire controls 114, 116 is transmitted into tension in the steering wires in order to control the bending of the steering section 18.

The hand controller includes control switches 120, 122, 124. In this embodiment, these are simple on-off electronic control switches.

The system is adapted to provide a flow of irrigation liquid to the region of tissue of interest from the base unit 12, along the umbilical section 16, through the hand controller 14 and along the insertion section 18. The base unit has an irrigation liquid valve (not shown). The irrigation liquid valve controls the flow of irrigation liquid. Electronic control switch 120 in turn controls the irrigation liquid valve. Therefore, in use, the operator can control the irrigation liquid (e.g. for irrigating the site of interest and/or cleaning the lens of the imaging chip) by pressing switch 120, i.e. the operation is carried out at the hand controller. However, the hand controller can still have the simple design indicated in Fig. 19 and so be economically manufactured and so suitable for single use before disposal.

The system is also adapted to provide a flow of insufflation gas to the region of tissue of interest from the base unit 12, along the umbilical section 16, through the hand controller 14 and along the insertion section 18. The base unit has an insufflation gas valve (not shown). The insufflation gas valve controls the flow of insufflation gas. Electronic control switch 122 in turn controls the insufflation gas valve. The operator can therefore control the insufflation gas by pressing switch 122, i.e. the operation is carried out at the hand controller.

The system is also adapted to provide suction to the region of tissue of interest, via the insertion section 18 and vacuum source 36. The base unit 12 has a suction control valve in the form of a pinch valve 40 operating on vacuum line 38 as described above. The hand controller has electronic suction control switch 124. Operation of the electronic suction control switch 124 at the hand controller controls the suction control valve 40 to control the provision of suction to the region of tissue of interest.

Known imaging endoscopes control the delivery of insufflation gas, irrigation liquid and suction using mechanical valves in the hand controller. These valves are referred to as "trumpet" type valves in view of their similarity in appearance and operation to valve keys on a trumpet. In such known endoscopes, the insufflation gas, irrigation liquid and suction are supplied from a base unit at respective suitable fixed pressures. The timing of delivery and the rate of delivery to the distal end of the endoscope is then controlled by the user operating the mechanical valves at the hand controller.

However, these mechanical valves are expensive to manufacture as they must be small enough to be operated by a finger, be regularly subject to high level disinfection. As will be understood, the operate in an analogue manner (allowing different flow rates of insufflation gas and irrigation liquid, for example, based on how far they are pressed by the user).

If the hand controller of embodiments of the invention were to require such mechanical valves, the cost of the hand controller (and thus of all of the disposable single use components of the endoscope system) would be prohibitively high.

Accordingly, it is preferred to have the valves controlling the insufflation gas, irrigation liquid and suction not in the hand controller but instead in the base unit. These valves physically control the timing of delivery and the rate of delivery of the insufflation gas, irrigation liquid and suction. However, these valves are in turn preferably controlled by corresponding electronic switches on the hand controller. Suitable electrical connections are provided between the hand controller and the base unit via the umbilical section.

The electronic switches may provide simple on-off control of the valves in the base unit. Alternatively, they may be operable digitally to provide at least partial analogue emulation control of the valves in the base unit. Suitable control methodologies will be known to the skilled person, but can be implemented via any manner suitable to an operator of the endoscope system, such as the length of time of depression of a switch, or the number of depressions of the switch, or speed of depressions of the switch, giving rise to a desired flow rate of the insufflation gas or irrigation liquid for example.

As will be understood, the concept of the use of electronic switches at the hand controller, controlling respective valves in the base unit, opens up an array of possibilities for operation of the endoscope system. The valves in the base unit can be designed to operate more efficiently and to operate digitally and in analogue emulation. There is great flexibility of design in this change: additional switches can be easily incorporated in the hand controller for new functions without increasing the complexity of the design of the disposable hand controller. The electronic switches can be configured to operate independently and/or in combinations of two or more through juxtaposition, depending on the operating features intended for the product. The electronic nature of the switches means that the operation of specific desirable functions can be set and tuned by using different software programming functions.

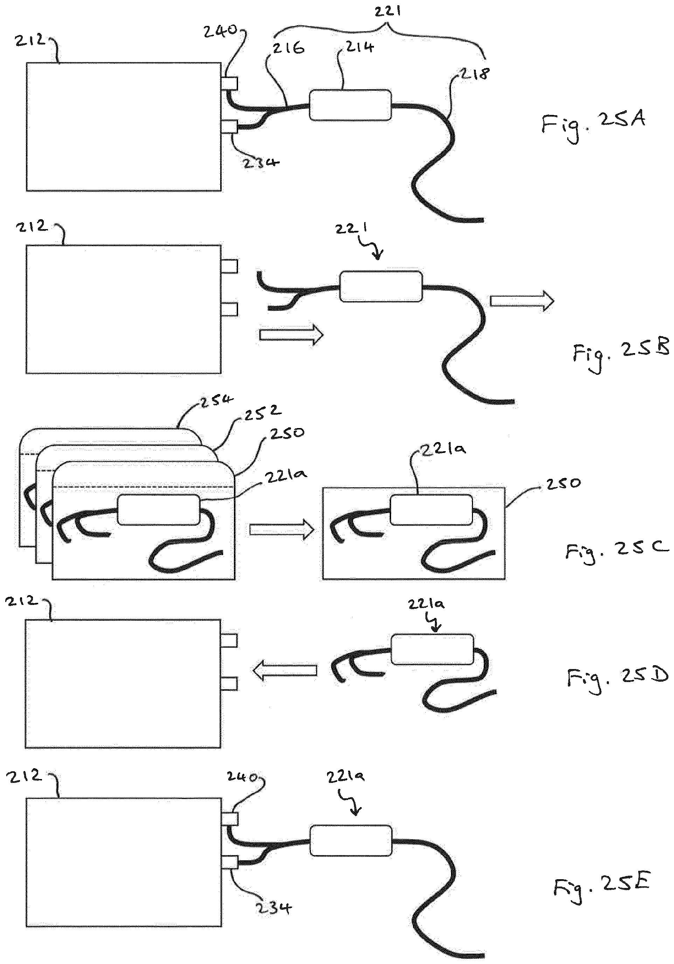

Figs. 25A-25E show schematically the process for swapping replacement interchangeable endoscope components according to an embodiment of the invention, with Figs. 25A-25E intended to be understood in sequence. Fig. 25A shows a starting condition, in which base unit 212 is attached to umbilical section 216 via connectors 234, 240. Umbilical section 216 leads to hand controller 214 which in turn leads to insertion section 218. Together, insertion section 216, hand controller 214 and insertion section 218 may be understood as an interchangeable endoscope component 221.

After an endoscopy procedure is carried out on a subject using interchangeable endoscope component 221, in order to prepare for another endoscopy procedure to be carried out on another subject, it is necessary to remove the interchangeable endoscope component 221 from the base unit. This is done by detaching the umbilical section 216 from the connectors 234, 240 on the base unit. The used interchangeable endoscope component 221 is then disposed of, as indicated in Fig. 25B.

There are provided several sealed container 250, 252, 254, each containing a respective interchangeable endoscope component 221a. These interchangeable endoscope components may be substantially identical. They are all in sterile condition in their respective sealed containers, which may be any suitable medical grade packaging. In Fig. 25C, container 250 is opened, allowing access to interchangeable endoscope component 221a.

In Fig. 25D, interchangeable endoscope component 221a is then attached to the base unit 212 using connector 234, 240, to provide an endoscopy system ready for use.

As illustrated in Figs. 25A-25E, a single reusable base unit can be used with several interchangeable identical endoscope components (comprising the umbilical section, hand controller and insertion section). One of the primary reasons for adopting this approach is that it is then possible to provide the interchangeable endoscope components in sterile condition at the time of attachment to the base unit. After a single use, the interchangeable endoscope component is intended to be detached from the base unit, disposed of, and the next interchangeable endoscope component, in sterile condition, attached to the base component ready for use. However, it is then desirable to ensure that one of the used (and therefore non-sterile) interchangeable endoscope components cannot be re-attached to the base unit for a subsequent use. Such re-attachment may be done through error or as a deliberate act. In order to provide a barrier to this occurrence, the system is designed in order to prevent the base unit from servicing an interchangeable endoscope component that has been previously attached and used on the same base unit. This can be achieved by using a single use confirmation system. The base unit has a first radio frequency identification means and the umbilical section has a second radio frequency identification means, the single use confirmation system permitting the imaging endoscopy system to operate only when it confirms, based on interaction between the first and second radio frequency identification means that the umbilical section has not previously been used with the base unit.

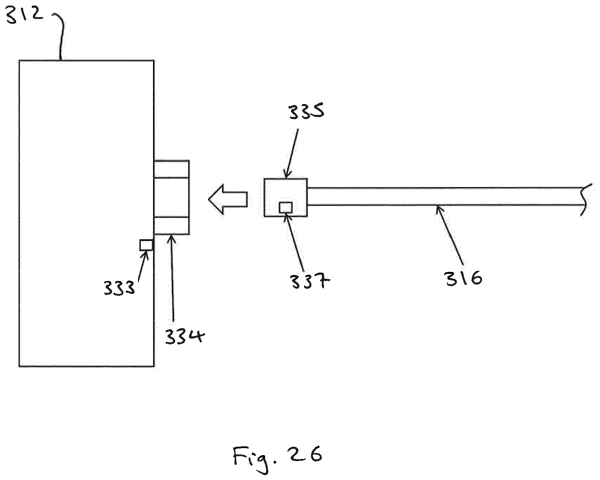

Fig. 26 shows a schematic view of the base unit 312 at the time of attachment of umbilical section 316 (the vacuum line is not shown, for simplicity). The hand controller and the insertion section are not shown. The base unit 312 provides a female connector 334 and the umbilical section 316 has a male connector 335. These connectors may be of the quick-connector type, allowing easy attachment and detachment and allowing electrical and fluid connection between the base unit and the umbilical section. The connector 335 on the umbilical section 316 has an active RFID or BLE component 337. The base unit 312, at or close to the female connector 334, has a corresponding active RFID or BLE detector 333. Such active RFID and BLE systems provide reliable operation and can be implemented using established standard communications protocols.

Given that the base unit 312 provides electrical power, irrigation liquid and insufflation gas to the insertion section, via the umbilical section and hand controller, the system can be rendered inoperable for an endoscopic procedure if one, more than one or all of supply of electrical power, irrigation liquid and insufflation gas are denied from being provided to the umbilical section. Such denial occurs when the single use confirmation system detects that the umbilical section has previously been used with the base unit. This is registered by the previous interaction of the active RFID or BLE component 337 and the corresponding active RFID or BLE detector 333. This renders the imaging endoscopy system inoperable and provides a substantial barrier to the accidental or deliberate re-use of the disposable components of the imaging endoscopy system.

In preferred embodiments of the invention, the hand controller uses rotational drive wheels connected to the steering section at the distal end of the insertion section. Steering is achieved using steering wires respectively coupled to the rotational drive wheels. The steering wires comprise Bowden cables. The outer constant length cable sheaths are connected proximally to the hand controller and distally at the proximal link of the steering section in four places. These four connection locations are equiangularly spaced around the insertion section of the endoscope, when viewed along the axis of the insertion section of the endoscope, for example at positions corresponding to 90°, 180°, 270° and 360°. The steering wires, which pass through the constant length cable sheaths, pass through the bending section and are connected to the distal link of the steering section and similarly equiangularly spaced at 90°, 180°, 270° and 360° in line with the relative distal ends of the cable sheaths. Proximally the drive cables are wrapped around and either connected to or frictionally gripped by the rotational drive wheels within the hand controller. For each axis of bending there is a respective drive wheel, which, when turned in either clockwise or counter clockwise pulls one of the steering wires and cause movement of the corresponding steering wire at 180° radially to it at the distal link. This has the effect of bending the steering section until rotation of the drive wheel is stopped. Accordingly, for the 90° and 270° steering wires, these form a first pair in the sense that a length change of one is accommodated by a corresponding length change in the other and they direct bending of the steering section in a first plane. For the 180° and 360° steering wires, these form a second pair in the sense that a length change of one is accommodated by a corresponding length change in the other and they direct bending of the steering section in a second plane, perpendicular to the first plane. The first pair of steering wires may be formed from a continuous steering wire looped around the first rotational drive wheel. Similarly, the second pair of steering wires may be formed from a continuous steering wire looped around the second rotational drive wheel. This improves the simplicity of the design.

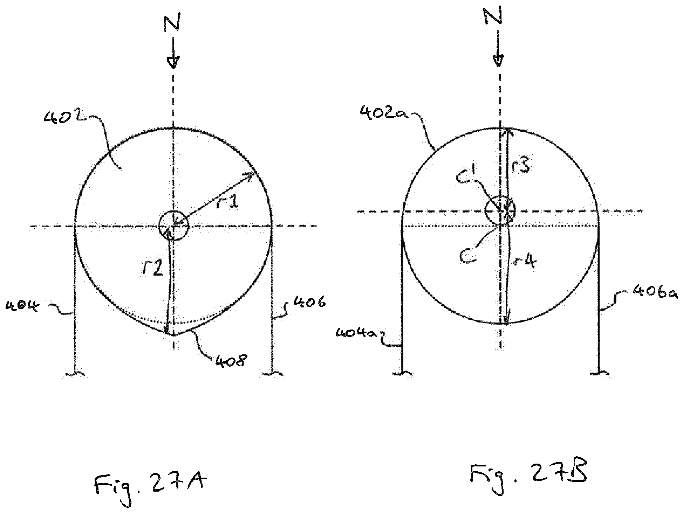

Fig. 27A shows a first example of a rotational drive wheel providing non-linear length change of the steering wires per angle of rotation. Rotational drive wheel 402 has a continuous wire wrapped around one half, the wire extending distally from the rotational drive wheel 402 to form a pair of steering wires 404, 406. Rotational drive wheel 402 has a non-circular shape, in the sense that it has a part-circular form, of radius r1 for the majority of its circumference and centred on neutral position N, but has a cam formation 408 of radius r2 greater than r1 at 180° from neutral position N. Rotation of the rotational drive wheel therefore provides substantially linear length change of the steering wires 404, 406 until the steering wire contacts the cam formation 408. Further rotation in the same direction causes greater length change per unit angle of rotation of the rotational drive wheel.

Fig. 27B shows a second example of a rotational drive wheel providing non-linear length change of the steering wires per angle of rotation. Rotational drive wheel 402a has a continuous wire wrapped around one half, the wire extending distally from the rotational drive wheel 402a to form a pair of steering wires 404a, 406a. Rotational drive wheel 402a has a circular shape, but is mounted for rotation about an axis C' which is disposed proximally of the geometric centre C of the rotational drive wheel. The rotational drive wheel 402a is therefore eccentrically mounted. The rotational drive wheel 402a is centred on neutral position N, at which point the distance from the circumference to the axis C' is r3. At a position 180° from neutral position N, the distance from the circumference to the axis C' is r4. Rotation of the rotational drive wheel therefore provides a gradually increasing rate of change of length of the steering wires 404a, 406a until the steering wire contacts the circumference of the rotational drive wheel at position 180° from neutral position N.

Using the embodiments shown in Figs. 27A or 27B, it is possible to have fine control over the bending of the steering section when close to the neutral position N, which corresponds to the steering section being straight in the plane of bending controlled by the rotational drive wheel. It is preferred that fine control is possible for bends of up to 15° for example. For greater bending angles, it is preferred that the gearing provided by the shape or mounting of the rotational drive wheels (described in relation to Figs. 27A or 27B) is harnessed to provide greater bending per angle of rotation of the rotational drive wheels.

The present design concept for the interchangeable endoscope hand controller uses a rotational drive wheel connected to the bending section of the insertion tube. These connections comprise 'Bowden' cables. The outer 'constant length' cable sheaths are connected proximally to the inside of the interchangeable hand controller and distally at the onto the proximal link of the bending section in 4 places, radially at 90°, 180°, 270° and 360°. The drive cables, which pass through the 'constant length' cable sheaths, pass through the bending section and are connected to the distal link of the bending section radially at 90°, 180°, 270° and 360° in line with the relative distal ends of the 'constant length' cable sheaths. Proximally the drive cables are connected to the rotational drive wheels within the interchangeable hand controller. For each axis of bending there is one drive wheel, which, when turned in either clockwise or counter clockwise will pull one of the drive cables and allow free movement of the corresponding drive cable at 180° radially to it at the distal link. This has the effect of bending the bending section of the interchangeable endoscope insertion tube until rotation is stopped. In order to make very small bending of the bending section very fine control of the tip must be exercised by the user. In order to make operation of the rotational drive wheels easier when small bends (less than 15°) the present design concept uses a cam profile on the cable drive wheel and/or an eccentric position for the drive pivot relative to the drive wheel cable driving interface.

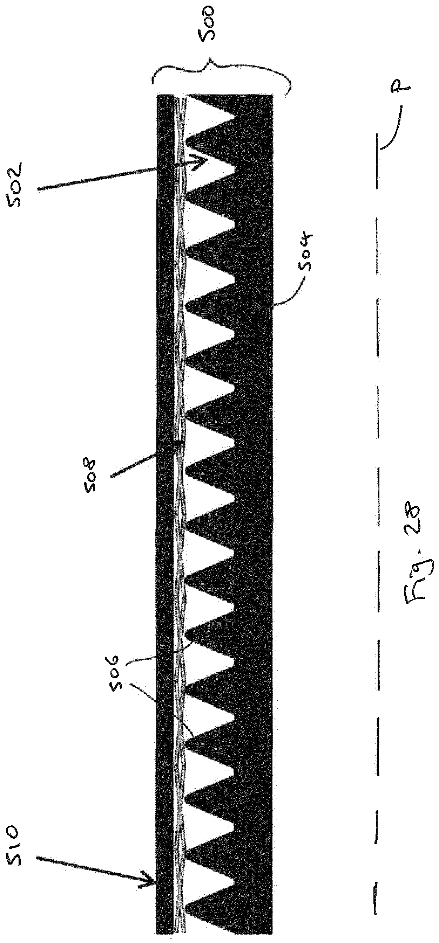

Fig. 28 shows a partial cross sectional view of an insertion section for use with an embodiment of the invention. An insertion section has a core part and an outer part, the outer part surrounding the core part. Fig. 28 shows only one side of the outer part 500 - the core part and the other side of the outer part are not shown.

The outer part 500 has a ridged inner tube 502. Specifically, an outer face of the ridged inner tube has a circumferential ridged configuration. That is, the ridges extend around the circumference of the inner tube 502. Preferably, they extend in a direction substantially perpendicular to the principal axis P of the insertion section. An inner face 504 of the ridged inner tube has a non-ridged configuration. Accordingly, the inner face is smooth. In cross section, as shown in Fig. 28, the ridged inner tube has a regular arrangement of peaks 506.

An outer reinforcing construction is provided around the ridged inner tube 502. The outer reinforcing construction comprises a braided fibre layer 508, overlying the ridged inner tube 502. The peaks are shaped and dimensioned to ensure that the braided fibre layer 508 it in contact only with the upper surfaces of the peaks. The outer reinforcing construction further comprises a smooth outer layer 510, overlying the braided fibre layer 508.

It is found that this construction provides better performance that that reported above in relation to a conventional reusable endoscope. That is, an approximately 13.00mm outer diameter (OD) insertion section can be bent to an inner radius of less than 90mmand can bear torsional loads of better than about 0.1 deg/Nm².

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention.

## Claims

1. A method for assembling an imaging endoscopy system for use in gastroscopy, the imaging endoscopy system comprising
a base unit;
a hand controller;
an umbilical section releasably connecting the base unit and the hand controller;
an insertion section having a proximal end connected to the hand controller and a distal end for insertion into a subject, the distal end having a steering section and a distal tip,
wherein:
the hand controller includes two rotational drive wheels for controlling bending of the steering section;
the distal tip includes a light source for illumination of a region of tissue of interest and an imaging chip for imaging the region of tissue of interest;
the insertion section includes four steering wires connecting the rotational drive wheels of the hand controller and the steering section, the steering section having two axes of bending;
the insertion section further including an electrical conductor for providing electrical power to the light source and an electrical conductor for conducting electrical signals from the imaging chip to the proximal end of the insertion section;
the insertion section further including lumens adapted to:
deliver insufflation gas to the distal tip;
deliver irrigation liquid to the distal tip;
provide suction at the distal tip;
provide a conduit for at least one surgical tool to be inserted along the insertion section to the distal tip;
wherein the method comprises:
disconnecting a used umbilical section from the base unit and disposing of said used umbilical section, along with a connected used hand controller and insertion section;
opening at least one sealed container containing:
a hand controller, in sterile condition;
an umbilical section, in sterile condition; and
an insertion section, in sterile condition, and
connecting the sterile umbilical section to the base unit to provide an imaging endoscopy system ready for use.

2. A method according to claim 1 wherein the hand controller has an imaging operation switch, adapted to control the system to record at least one of moving images and still images.

3. A method according to claim 1 or claim 2 wherein the insertion section has a core part and an outer part, the outer part surrounding the core part, the core part being formed as an extruded member having a cross sectional shape providing at least two integral internal lumens extending along the interior of the core part from the proximal end to the distal end, the insertion section further comprising said steering wires, extending from the proximal end for connection to the hand controller to the distal tip, the steering wires being located between the core part and the outer part of the insertion section, wherein the steering wires have sheaths each defining an axis of constrained movement for each respective steering wire, and wherein the steering wires extend through the steering section to the distal tip and the sheaths of the steering wires do not extend through the steering section.

4. A method according to claim 3 wherein the steering section has a series of links pivotably connected to each other, the links arranged to cooperate to permit controlled bending of the steering section, and optionally wherein pivotable connections between each link provides a limit to the range of relative movement between adjacent links, and optionally wherein the links of the steering section have apertures dimensioned to receive and conduct the steering wires.

5. A method according to claim 3 or claim 4 wherein the core part extends through the steering section to the distal tip.

6. A method according to any one of claims 1 to 5 wherein the distal tip includes a distal tip housing which is integrally formed from light-transmissive material, and optionally wherein the distal tip housing is integrally formed from plastics material.

7. A method according to any one of claims 1 to 6 wherein, when the umbilical section is detached from the base unit and the hand controller and insertion section are immersed in water at room temperature and pressure, the hand controller and/or the insertion section is water permeable to permit ingress of water into the steering channels in the insertion section.

8. An imaging endoscopy kit for assembling an imaging endoscopy system for use in gastroscopy, the imaging endoscopy system assembled from the kit comprising:
a base unit;
a hand controller;
an umbilical section releasably connecting the base unit and the hand controller;
an insertion section having a proximal end connected to the hand controller and a distal end for insertion into a subject, the distal end having a steering section and a distal tip,
wherein:
the hand controller includes two rotational drive wheels for controlling bending of the steering section;
the distal tip includes a light source for illumination of a region of tissue of interest and an imaging chip for imaging the region of tissue of interest;
the insertion section includes four steering wires connecting the rotational drive wheels of the hand controller and the steering section, the steering section having two axes of bending;
the insertion section further including an electrical conductor for providing electrical power to the light source and an electrical conductor for conducting electrical signals from the imaging chip to the proximal end of the insertion section;
the insertion section further including lumens adapted to:
deliver insufflation gas to the distal tip;
deliver irrigation liquid to the distal tip;
provide suction at the distal tip;
provide a conduit for at least one surgical tool to be inserted along the insertion section to the distal tip;
wherein the imaging endoscopy kit comprises:
the base unit; and
a plurality of sealed containers, together containing:
a plurality of said hand controllers, in sterile condition;
a plurality of said umbilical sections, in sterile condition; and
a plurality of said insertion sections, in sterile condition.

9. A kit according to claim 8 wherein the hand controller has an imaging operation switch, adapted to control the system to record at least one of moving images and still images.

10. A kit according to claim 8 or claim 9 wherein the insertion section has a core part and an outer part, the outer part surrounding the core part, the core part being formed as an extruded member having a cross sectional shape providing at least two integral internal lumens extending along the interior of the core part from the proximal end to the distal end, the insertion section further comprising said steering wires, extending from the proximal end for connection to the hand controller to the distal tip, the steering wires being located between the core part and the outer part of the insertion section, wherein the steering wires have sheaths each defining an axis of constrained movement for each respective steering wire, and wherein the steering wires extend through the steering section to the distal tip and the sheaths of the steering wires do not extend through the steering section.

11. A kit according to claim 10 wherein the steering section has a series of links pivotably connected to each other, the links arranged to cooperate to permit controlled bending of the steering section, and optionally wherein pivotable connections between each link provides a limit to the range of relative movement between adjacent links, and optionally wherein the links of the steering section have apertures dimensioned to receive and conduct the steering wires.

12. A kit according to any one of claims 8 to 11 wherein the core part extends through the steering section to the distal tip.

13. A kit according to any one of claims 8 to 12 wherein the distal tip includes a distal tip housing which is integrally formed from light-transmissive material, and optionally wherein the distal tip housing is integrally formed from plastics material.

14. A kit according to any one of claims 8 to 13 wherein, when the umbilical section is detached from the base unit and the hand controller and insertion section are immersed in water at room temperature and pressure, the hand controller and/or the insertion section is water permeable to permit ingress of water into the steering channels in the insertion section.

## Patentansprüche

1. Verfahren zum Zusammenbau eines bildgebenden Endoskopiesystems zur Verwendung bei einer Gastroskopie, wobei das bildgebende Endoskopiesystem Folgendes umfasst:
eine Basiseinheit;
eine Handsteuerung;
einen Kabelabschnitt, der die Basiseinheit und die Handsteuerung lösbar verbindet;
einen Einführungsabschnitt, der ein proximales Ende, das mit der Handsteuerung verbunden ist, und ein distales Ende zum Einführen in ein Individuum aufweist, wobei das distale Ende einen Steuerabschnitt und eine distale Spitze aufweist,
wobei:
die Handsteuerung zwei Drehantriebsräder zum Steuern der Krümmung des Steuerabschnitts aufweist;
die distale Spitze eine Lichtquelle zur Beleuchtung eines Gewebebereichs von Interesse und einen bildgebenden Chip zum Abbilden des Gewebebereichs von Interesse aufweist;
der Einführungsabschnitt vier Steuerdrähte aufweist, die die Drehantriebsräder der Handsteuerung und den Steuerabschnitt verbinden, wobei der Steuerabschnitt zwei Krümmungsachsen aufweist;
der Einführungsabschnitt ferner einen elektrischen Leiter zum Bereitstellen elektrischer Leistung an der Lichtquelle und einen elektrischen Leiter zum Leiten elektrischer Signale von dem bildgebenden Chip an das proximale Ende des Einführungsabschnitts aufweist;
wobei der Einführungsabschnitt ferner Lumen umfasst, die ausgelegt sind, um:
Insufflationsgas an die distale Spitze zuzuführen;
Spülflüssigkeit an die distale Spitze zuzuführen;
Absaugwirkung an der distalen Spitze bereitzustellen;
einen Kanal für zumindest ein chirurgisches Instrument bereitzustellen, das entlang des Einführungsabschnitts bis zur distalen Spitze einzuführen ist;
wobei das Verfahren Folgendes umfasst:
Abkoppeln eines benutzten Kabelabschnitts von der Basiseinheit und Entsorgen des benutzten Kabelabschnitts zusammen mit einer verbundenen benutzten Handsteuerung und eines Einführungsabschnitts;
Öffnen von zumindest einem dicht verschlossenen Behälter, der Folgendes enthält:
eine Handsteuerung in sterilem Zustand;
einen Kabelabschnitt in sterilem Zustand; und
einen Einführungsabschnitt in sterilem Zustand; und
Verbinden des sterilen Kabelabschnitts mit der Basiseinheit, um ein gebrauchsfertiges bildgebendes Endoskopiesystem bereitzustellen.

2. Verfahren nach Anspruch 1, wobei die Handsteuerung einen bildgebenden Betriebsschalter umfasst, der ausgelegt ist, um das System zu steuern, um zumindest eines aus Bewegtbildern und Standbildern aufzunehmen.

3. Verfahren nach Anspruch 1 oder Anspruch 2, wobei der Einführungsabschnitt einen Kernteil und einen Hüllenteil aufweist, wobei der Hüllenteil den Kernteil umgibt, wobei der Kernteil als extrudiertes Element ausgebildet ist, das eine Querschnittsform aufweist, die zumindest zwei integrierte interne Lumen bereitstellt, die sich entlang des Inneren des Kernteils vom proximalen Ende bis zum distalen Ende erstrecken, wobei der Einführungsabschnitt ferner die Steuerdrähte umfasst, die sich vom proximalen Ende zur Verbindung mit der Handsteuerung bis zur distalen Spitze erstrecken, wobei sich die Steuerdrähte zwischen dem Kernteil und dem Hüllenteil des Einführungsabschnitts befinden, wobei die Steuerdrähte Umhüllungen aufweisen, die jeweils eine Achse einer eingeschränkten Bewegung für jeden entsprechenden Steuerdraht definieren, und wobei sich die Steuerdrähte durch den Steuerabschnitt bis zur distalen Spitze erstrecken und sich die Umhüllungen der Steuerdrähte nicht durch den Steuerabschnitt erstrecken.

4. Verfahren nach Anspruch 3, wobei der Steuerabschnitt eine Reihe von Verbindungselementen aufweist, die schwenkbar miteinander verbunden sind, wobei die Verbindungselemente angeordnet sind, um zusammenzuwirken, um eine kontrollierte Krümmung des Steuerabschnitts zuzulassen, und wobei schwenkbare Verbindungen zwischen jedem Verbindungselement gegebenenfalls eine Begrenzung des Bereichs der relativen Bewegung zwischen benachbarten Verbindungselementen bereitstellen, und wobei die Verbindungselemente des Steuerabschnitts gegebenenfalls Öffnungen aufweisen, die so bemessen sind, dass sie Steuerdrähte aufnehmen und führen.

5. Verfahren nach Anspruch 3 oder Anspruch 4, wobei sich der Kernteil durch den Steuerabschnitt bis zur distalen Spitze erstreckt.

6. Verfahren nach einem der Ansprüche 1 bis 5, wobei die distale Spitze ein Gehäuse der distalen Spitze umfasst, das einstückig aus lichtdurchlässigem Material ausgebildet ist, und wobei das Gehäuse der distalen Spitze gegebenenfalls einstückig aus einem Kunststoffmaterial ausgebildet ist.

7. Verfahren nach einem der Ansprüche 1 bis 6, wobei, wenn der Kabelabschnitt von der Basiseinheit gelöst ist, und die Handsteuerung und der Einführungsabschnitt bei Raumtemperatur und -druck in Wasser eingetaucht werden, die Handsteuerung und/oder der Einführungsabschnitt wasserdurchlässig sind, um das Eintreten von Wasser in die Steuerkanäle in dem Einführungsabschnitt zu ermöglichen.

8. Bildgebendes Endoskopieset zum Zusammenbau eines bildgebenden Endoskopiesystems zur Verwendung bei einer Gastroskopie, wobei das aus dem Set zusammengebaute bildgebende Endoskopiesystem Folgendes umfasst:
eine Basiseinheit;
eine Handsteuerung;
einen Kabelabschnitt, der die Basiseinheit und die Handsteuerung lösbar verbindet;
einen Einführungsabschnitt, der ein proximales Ende, das mit der Handsteuerung verbunden ist, und ein distales Ende zum Einführen in ein Individuum aufweist, wobei das distale Ende einen Steuerabschnitt und eine distale Spitze aufweist,
wobei:
die Handsteuerung zwei Drehantriebsräder zum Steuern der Krümmung des Steuerabschnitts aufweist;
die distale Spitze eine Lichtquelle zur Beleuchtung eines Gewebebereichs von Interesse und einen bildgebenden Chip zum Abbilden des Gewebebereichs von Interesse aufweist;
der Einführungsabschnitt vier Steuerdrähte aufweist, die die Drehantriebsräder der Handsteuerung und den Steuerabschnitt verbinden, wobei der Steuerabschnitt zwei Krümmungsachsen aufweist;
der Einführungsabschnitt ferner einen elektrischen Leiter zum Bereitstellen elektrischer Leistung an der Lichtquelle und einen elektrischen Leiter zum Leiten elektrischer Signale von dem bildgebenden Chip an das proximale Ende des Einführungsabschnitts aufweist;
wobei der Einführungsabschnitt ferner Lumen umfasst, die ausgelegt sind, um:
Insufflationsgas an die distale Spitze zuzuführen;
Spülflüssigkeit an die distale Spitze zuzuführen;
Absaugwirkung an der distalen Spitze bereitzustellen;
einen Kanal für zumindest ein chirurgisches Instrument bereitzustellen, das entlang des Einführungsabschnitts bis zur distalen Spitze einzuführen ist;
wobei das bildgebende Endoskopieset Folgendes umfasst:
die Basiseinheit; und
eine Vielzahl von dicht verschlossenen Behältern, die zusammen Folgendes enthalten:
eine Vielzahl der Handsteuerungen in sterilem Zustand;
eine Vielzahl der Kabelabschnitte in sterilem Zustand; und
eine Vielzahl der Einführungsabschnitte in sterilem Zustand.

9. Set nach Anspruch 8, wobei die Handsteuerung einen bildgebenden Betriebsschalter umfasst, der ausgelegt ist, um das System zu steuern, um zumindest eines aus Bewegtbildern und Standbildern aufzunehmen.

10. Set nach Anspruch 8 oder Anspruch 9, wobei der Einführungsabschnitt einen Kernteil und einen Hüllenteil aufweist, wobei der Hüllenteil den Kernteil umgibt, wobei der Kernteil als extrudiertes Element ausgebildet ist, das eine Querschnittsform aufweist, die zumindest zwei integrierte interne Lumen bereitstellt, die sich entlang des Inneren des Kernteils vom proximalen Ende bis zum distalen Ende erstrecken, wobei der Einführungsabschnitt ferner die Steuerdrähte umfasst, die sich vom proximalen Ende zur Verbindung mit der Handsteuerung bis zur distalen Spitze erstrecken, wobei sich die Steuerdrähte zwischen dem Kernteil und dem Hüllenteil des Einführungsabschnitts befinden, wobei die Steuerdrähte Umhüllungen aufweisen, die jeweils eine Achse einer eingeschränkten Bewegung für jeden entsprechenden Steuerdraht definieren, und wobei sich die Steuerdrähte durch den Steuerabschnitt bis zur distalen Spitze erstrecken und sich die Umhüllungen der Steuerdrähte nicht durch den Steuerabschnitt erstrecken.

11. Set nach Anspruch 10, wobei der Steuerabschnitt eine Reihe von Verbindungselementen aufweist, die schwenkbar miteinander verbunden sind, wobei die Verbindungselemente angeordnet sind, um zusammenzuwirken, um eine kontrollierte Krümmung des Steuerabschnitts zuzulassen, und wobei schwenkbare Verbindungen zwischen jedem Verbindungselement gegebenenfalls eine Begrenzung des Bereichs der relativen Bewegung zwischen benachbarten Verbindungselementen bereitstellen, und wobei die Verbindungselemente des Steuerabschnitts gegebenenfalls Öffnungen aufweisen, die so bemessen sind, dass sie Steuerdrähte aufnehmen und führen.

12. Set nach einem der Ansprüche 8 bis 11, wobei sich der Kernteil durch den Steuerabschnitt bis zur distalen Spitze erstreckt.

13. Set nach einem der Ansprüche 8 bis 12, wobei die distale Spitze ein Gehäuse der distalen Spitze umfasst, das einstückig aus lichtdurchlässigem Material ausgebildet ist, und wobei das Gehäuse der distalen Spitze gegebenenfalls einstückig aus einem Kunststoffmaterial ausgebildet ist.

14. Set nach einem der Ansprüche 8 bis 13, wobei, wenn der Kabelabschnitt von der Basiseinheit gelöst ist, und die Handsteuerung und der Einführungsabschnitt bei Raumtemperatur und -druck in Wasser eingetaucht werden, die Handsteuerung und/oder der Einführungsabschnitt wasserdurchlässig sind, um das Eintreten von Wasser in die Steuerkanäle in dem Einführungsabschnitt zu ermöglichen.

## Revendications

1. Procédé pour assembler un système d'endoscopie par imagerie destiné à être utilisé en gastroscopie, le système d'endoscopie par imagerie comprenant :
une unité de base ;
une commande manuelle ;
une section ombilicale raccordant, de manière amovible, l'unité de base et la commande manuelle ;
une section d'insertion ayant une extrémité proximale raccordée à la commande manuelle et une extrémité distale pour insertion dans un patient, l'extrémité distale avant une section de direction et une pointe distale,
dans lequel :
la commande manuelle comprend deux roues d'entraînement rotatives pour contrôler la flexion de la section de direction ;
la pointe distale comprend une source de lumière pour l'éclairage d'une région de tissu d'intérêt et une puce d'imagerie pour imager la région du tissu d'intérêt ;
la section d'insertion comprend quatre fils de direction raccordant les roues d'entraînement rotatives de la commande manuelle et la section de direction, la section de direction ayant deux axes de flexion ;
la section d'insertion comprenant en outre un conducteur électrique pour fournir l'énergie électrique à la source de lumière et un conducteur électrique pour conduire les signaux électriques de la puce d'imagerie à l'extrémité proximale de la section d'insertion ;
la section d'insertion comprenant en outre des lumières adaptées pour :
distribuer du gaz d'insufflation à la pointe distale ;
distribuer du liquide d'irrigation à la pointe distale ;
fournir l'aspiration au niveau de la pointe distale ;
fournir un conduit pour au moins un outil chirurgical à insérer le long de la section d'insertion dans la pointe distale ;
dans lequel le procédé comprend les étapes consistant à :
déconnecter une section ombilicale usagée de l'unité de base et jeter ladite section ombilicale usagée, conjointement avec une commande manuelle usagée raccordée et avec la section d'insertion ;
ouvrir au moins un récipient étanche contenant :
une commande manuelle, en condition stérile ;
une section ombilicale, en condition stérile ; et
une section d'insertion, en condition stérile ; et
raccorder la section ombilicale stérile à l'unité de base pour fournir un système d'endoscopie par imagerie prêt à l'emploi.

2. Procédé selon la revendication 1, dans lequel la commande manuelle a un commutateur d'opération d'imagerie adapté pour commander le système afin qu'il enregistre au moins l'une parmi les images mobiles et les images immobiles.

3. Procédé selon la revendication 1 ou la revendication 2, dans lequel la section d'insertion a une partie de noyau et une partie externe, la partie externe entourant la partie de noyau, la partie de noyau étant formée comme un élément extrudé ayant une forme transversale fournissant au moins deux lumières internes solidaires s'étendant le long de l'intérieur de la partie de noyau de l'extrémité proximale à l'extrémité distale, la section d'insertion comprenant en outre lesdits fils de direction, s'étendant à partir de l'extrémité proximale pour le raccordement avec la commande manuelle jusqu'à la pointe distale, les fils de direction étant positionnés entre la partie de noyau et la partie externe de la section d'insertion, dans lequel les fils de direction ont des gaines définissant chacune un axe de mouvement contraint pour chaque fil de direction respectif, et dans lequel les fils de direction s'étendent par la section de direction jusqu'à la pointe distale et les gaines des fils de direction ne s'étendent pas à travers la section de direction.

4. Procédé selon la revendication 3, dans lequel la section de direction a une série de liaisons raccordées, de manière pivotante, entre elles, les liaisons étant agencées pour coopérer afin de permettre la flexion contrôlée de la section de direction, et facultativement dans lequel les raccordements pivotants entre chaque liaison, fournissent une limite à la plage de mouvement relatif entre les liaisons adjacentes, et facultativement dans lequel les liaisons de la section de direction ont des ouvertures dimensionnées pour recevoir et conduire les fils de direction.

5. Procédé selon la revendication 3 ou la revendication 4, dans lequel la partie de noyau s'étend par la section de direction jusqu'à la pointe distale.

6. Procédé selon l'une quelconque des revendications 1 à 5, dans lequel la pointe distale comprend un boîtier de pointe distale qui est formé, de manière solidaire, à partir d'un matériau de transmission de lumière, et facultativement dans lequel le bâti de pointe distale est formé, de manière solidaire, à partir de matière plastique.

7. Procédé selon l'une quelconque des revendications 1 à 6, dans lequel, lorsque la section ombilicale est détachée de l'unité de base et que la commande manuelle et la section d'insertion sont immergées dans l'eau à une température et une pression ambiante, la commande manuelle et/ou la section d'insertion est (sont) perméable(s) à l'eau pour permettre l'entrée d'eau dans les canaux de direction dans la section d'insertion.

8. Kit d'endoscopie par imagerie pour assembler un système d'endoscopie par imagerie destiné à être utilisé en gastroscopie, le système d'endoscopie par imagerie assemblé à partir du kit comprenant :
une unité de base ;
une commande manuelle ;
une section ombilicale raccordant, de manière amovible, l'unité de base et la commande manuelle ;
une section d'insertion ayant une extrémité proximale raccordée à la commande manuelle et une extrémité distale pour insertion dans un patient, l'extrémité distale avant une section de direction et une pointe distale,
dans lequel :
la commande manuelle comprend deux roues d'entraînement rotatives pour contrôler la flexion de la section de direction ;
la pointe distale comprend une source de lumière pour l'éclairage d'une région de tissu d'intérêt et une puce d'imagerie pour imager la région du tissu d'intérêt ;
la section d'insertion comprend quatre fils de direction raccordant les roues d'entraînement rotatives de la commande manuelle et la section de direction, la section de direction ayant deux axes de flexion ;
la section d'insertion comprenant en outre un conducteur électrique pour fournir l'énergie électrique à la source de lumière et un conducteur électrique pour conduire les signaux électriques de la puce d'imagerie à l'extrémité proximale de la section d'insertion ;
la section d'insertion comprenant en outre des lumières adaptées pour :
distribuer du gaz d'insufflation à la pointe distale ;
distribuer du liquide d'irrigation à la pointe distale ;
fournir l'aspiration au niveau de la pointe distale ;
fournir un conduit pour au moins un outil chirurgical à insérer le long de la section d'insertion dans la pointe distale ;
dans lequel le kit d'endoscopie par imagerie comprend :
l'unité de base ; et
une pluralité de boîtiers étanches, contenant ensemble :
une pluralité desdites commandes manuelles, en condition stérile ;
une pluralité desdites sections ombilicales, en condition stérile ; et
une pluralité desdites sections d'insertion, en condition stérile.

9. Kit selon la revendication 8, dans lequel la commande manuelle a un commutateur d'opération d'imagerie, adapté pour commander le système afin qu'il enregistre au moins l'une parmi les images mobiles et les images immobiles.

10. Kit selon la revendication 8 ou la revendication 9, dans lequel la section d'insertion a une partie de noyau et une partie externe, la partie externe entourant la partie de noyau, la partie de noyau étant formée comme un élément extrudé ayant une forme transversale fournissant au moins deux lumières internes solidaires s'étendant le long de l'intérieur de la partie de noyau à partir de l'extrémité proximale jusqu'à l'extrémité distale, la section d'insertion comprenant en outre lesdits fils de direction, s'étendant à partir de l'extrémité proximale pour le raccordement à la commande manuelle jusqu'à la pointe distale, les fils de direction étant positionnés entre la partie de noyau et la partie externe de la section d'insertion, dans lequel les fils de direction ont des gaines, définissant chacune un axe de mouvement contraint pour chaque fil de direction respectif, et dans lequel les fils de direction s'étendent par la section de direction jusqu'à la pointe distale et les gaines des fils de direction ne s'étendent pas à travers la section de direction.

11. Kit selon la revendication 10, dans lequel la section de direction a une série de liaisons raccordées, de manière pivotante, entre elles, les liaisons étant agencées pour coopérer afin de permettre la flexion contrôlée de la section de direction, et facultativement dans lequel les raccordements pivotants entre chaque liaison fournissent une limite à la plage de mouvement relatif entre les liaisons adjacentes, et facultativement dans lequel les liaisons de la section de direction ont des ouvertures dimensionnées pour recevoir et conduire les fils de direction.

12. Kit selon l'une quelconque des revendications 8 à 11, dans lequel la partie de noyau s'étend par la section de direction jusqu'à la pointe distale.

13. Kit selon l'une quelconque des revendications 8 à 12, dans lequel la pointe distale comprend un boîtier de pointe distale qui est formé, de manière solidaire, à partir d'un matériau de transmission de lumière, et facultativement dans lequel le boîtier de pointe distale est formé, de manière solidaire, à partir de matière plastique.

14. Kit selon l'une quelconque des revendications 8 à 13 dans lequel, lorsque la section ombilicale est détachée de l'unité de base et que la commande manuelle et la section d'insertion sont immergées dans l'eau à température et à pression ambiante, la commande manuelle et/ou la section d'insertion est (sont) perméable(s) à l'eau pour permettre l'entrée de l'eau dans les canaux de direction dans la section d'insertion.
